# EUROPEAN PATENT APPLICATION

(11) **EP 4 782 837 A1**
(43) Date of publication of application: **29.07.2026**
(21) Application number: 24868797.2
(22) Date of filing: 19.09.2024
(51) Int. Cl.: G01N 33/574, C07K 16/28

(54) **COMPOSITION FOR PREDICTING CETUXIMAB RESPONSIVENESS IN CANCER**

(30) Priority: 19.09.2023 KR 20230124853
(71) Applicant: Korea Institute of Science and Technology, Seoul 02792 (KR)
(72) Inventor: RYU, Ju Hee, Seoul 02792 (KR); KWON, Ick Chan, Seoul 02792 (KR); HONG, Seung Taek, Seoul 02792 (KR); SEONG, Yejin, Seoul 02792 (KR)
(74) Representative: Handley, Matthew Edward
(86) International application number: PCT/KR2024/096185
(87) International publication number: WO 2025/063823

(57) **Abstract**

The present invention relates to a composition for predicting the responsiveness to cetuximab in cancer, more specifically, a composition comprising a complex, wherein the complex comprises: a peptide represented by the amino acid sequence of SEQ ID NO: 1, which is cleaved by a lysosomal enzyme present in a cancer cell; a fluorophore and a quencher bound to the peptide; and cetuximab bound to the C-terminus of the peptide. The Cetux-probe of the present invention operates via the same mechanism of action as cetuximab, enabling rapid and accurate prediction of cetuximab's therapeutic efficacy and responsiveness. The activated fluorescence of the Cetux-probe can be used to measure EGFR degradation, and a strong linear correlation with cetuximab's cytotoxicity was observed in colorectal cancer cells and in mice bearing tumors. Furthermore, it was confirmed that the predictive capability of the activated fluorescence of the Cetux-probe is significantly higher than that of EGFR expression or KRAS mutation status. Therefore, the Cetux-probe of the present invention can be effectively utilized to predict responsiveness to cetuximab therapy by evaluating EGFR degradation.

## Description

### [TECHNICAL FIELD]

The present invention relates to a composition for predicting cetuximab responsiveness in cancer, and more specifically, to a composition for predicting cetuximab responsiveness comprising a complex (Cetux-probe), wherein the complex comprises: a peptide, which is cleaved by a lysosomal enzyme present in a cancer cell; a fluorophore and a quencher bound to the peptide; and cetuximab bound to the C-terminus of the peptide.

### [BACKGROUND ART]

Activation of the epidermal growth factor receptor (EGFR) plays a crucial role in the growth and aggressiveness of various cancers, including colorectal cancer, head and neck cancer, lung cancer, and pancreatic cancer. Therefore, EGFR is an important target in cancer therapy. Adding the EGFR-targeted antibody cetuximab to standard chemotherapy significantly improves treatment efficacy in patients with metastatic colorectal cancer. However, responsiveness to cetuximab varies considerably among individuals, and the clinical benefit appears limited to specific subgroups of colorectal cancer patients.

Early differentiation between responders and non-responders can minimize unnecessarily prolonged treatment, thereby avoiding significant limitations such as severe toxicity and high costs. Therefore, developing methods to predict responsiveness to anti-EGFR therapy is essential to maximize treatment outcomes. Consequently, recent research has primarily focused on predicting responsiveness to anti-EGFR therapy.

Currently, four EGFR monoclonal antibodies (cetuximab, panitumumab, necitumumab, and nimotuzumab) are commercially available, and several other EGFR monoclonal antibody drugs are in clinical trials. Initial clinical trials considered anti-EGFR therapy only for patients confirmed EGFR-positive via immunohistochemical staining. However, multiple studies have revealed that initial EGFR expression levels are not correlated with responsiveness to anti-EGFR therapy.

To date, KRAS mutation status remains the only biomarker usable to predict responsiveness to anti-EGFR therapy in combination with chemotherapy for colorectal cancer. KRAS-mutated colorectal cancers are considered unresponsive to cetuximab because the KRAS mutation leads to constitutive activation of downstream EGFR signaling.

However, the response rate to anti-EGFR therapy is also low at 40% in KRAS wild-type colorectal cancer patients, necessitating new and reliable approaches to predict responsiveness to anti-EGFR therapy (W. De Roock, et al., Ann. Oncol., 19:508-515, 2008).

Anti-EGFR antibodies exert antitumor activity through multiple mechanisms. They can bind to the extracellular domain of EGFR, blocking ligand binding and downstream signaling, and can bind to Fcy receptors, inducing antibody-dependent cellular cytotoxicity. Another important mechanism of action for anti-EGFR antibodies is inducing EGFR internalization and degradation. The internalization and degradation of activated receptors is an essential mechanism for downregulating growth-promoting signals within cells (H. Sunada, et al., Proc. Natl. Acad. Sci. U. S. A., 83:3825-3829, 1986).

As the importance of effective degradation of specific disease-related proteins like EGFR for disease treatment is emphasized, various targeted protein degradation strategies such as PROTAC (proteolysis-targeting chimera) and LYTAC (lysosome-targeting chimera) have recently garnered significant attention. Accumulating evidence indicates that the degree of EGFR degradation, rather than EGFR expression levels following EGFR-mediated internalization, is significantly correlated with the anticancer efficacy of anti-EGFR antibodies (Y. Okada, et al., Mol. Cancer Res., 15:1445-1454, 2017). Previous research results suggest that monitoring EGFR degradation following EGFR-mediated internalization could serve as a means to predict responsiveness to anti-EGFR therapy.

### [DISCLOSURE OF THE INVENTION]

### [TECHNICAL PROBLEM]

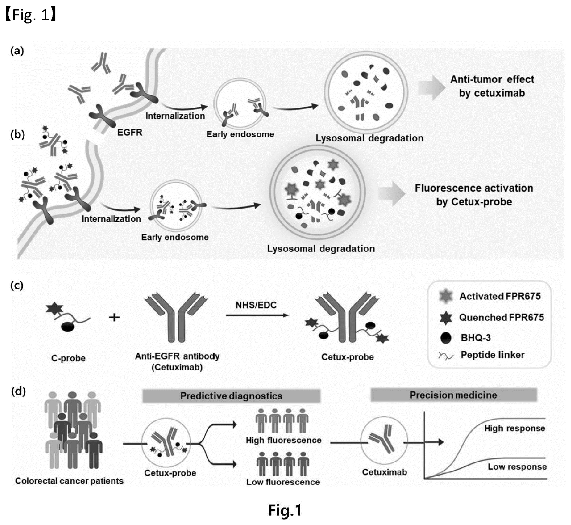

Accordingly, the present invention has developed a novel fluorescent probe, Cetux-probe, to evaluate EGFR degradation following anti-EGFR therapy. Specifically, the present inventors carefully investigated the mechanism by which anti-EGFR antibodies (cetuximab) induce EGFR degradation, and then prepared the Cetux-probe to function via the same mechanism as this therapeutic process. As shown in Fig. 1, cetuximab binds to EGFR and is internalized into the cell as a cetuximab:EGFR complex. This complex is transported to lysosomes via early endosomes, leading to EGFR proteolysis.

The present Cetux-probe was rationally designed to share this mechanism of action, binding to EGFR, being internalized into the cell, transported to lysosomes, and degraded. Just as the degradation of the cetuximab:EGFR complex in lysosomes enhances cetuximab's anticancer effect, the degradation of Cetux-probe:EGFR complex in lysosomes can activate the fluorescent signal.

Therefore, an objective of the present invention is to provide a composition for predicting responsiveness to cetuximab in cancer or for confirming the prognosis of the therapeutic efficacy of cetuximab.

Another objective of the present invention is to provide a method for providing information to predict responsiveness to cetuximab using the aforementioned composition.

Another objective of the present invention is to provide a composition for diagnosing EGFR-positive cancer.

### [TECHNICAL SOLUTION]

To achieve the aforementioned objectives, the present invention provides a composition for predicting cetuximab responsiveness or confirming the prognosis regarding cetuximab treatment efficacy, comprising a complex, wherein the complex comprises: a peptide represented by the amino acid sequence of SEQ ID NO: 1, which is cleaved by a lysosomal enzyme present in a cancer cell; a fluorophore and a quencher bound to the peptide; and cetuximab bound to the C-terminus of the peptide.

In a preferred embodiment of the present invention, the fluorophore may be bound to the N-terminus of the peptide, and the quencher may be bound to the epsilon-amino group of a lysine or arginine amino acid residue of the peptide.

In another preferred embodiment of the present invention, the fluorophore may be selected from the group consisting of fluorescein, FITC (fluorescein isothiocyanate), Oregon green, Texas red, Cy2, Cy3, Cy3B, Cy3.5, Cy5, Cy5.5, Cy7, indocarbocyanine, rhodamine, oxacarbocyanine, thiacarbocyanine, merocyanine, pyrodyloxazole, nitrobenzoxadiazole, benzoxadiazole, Nile red, Nile orange, acridine yellow, aumarine, crystal violet, and malachite green.

In another preferred embodiment of the present invention, the quencher may be selected from the group consisting of TAMRA (6-carboxytetramethylrhodamine), BHQ1 (black hole quencher 1), BHQ2 (black hole quencher 2), BHQ3 (black hole quencher 3), NFQ (nonfluorescent quencher), dabcyl, Eclipse, DDQ (Deep Dark Quencher), Blackberry Quencher, and Iowa black.

In another preferred embodiment of the present invention, the cancer may be epidermal growth factor receptor (EGFR)-positive cancer, and preferably EGFR-positive colorectal cancer.

In another preferred embodiment of the present invention, the complex can penetrate cancer cells and be cleaved by enzymes present within the lysosomes of the cancer cells, thereby relieving the quenching effect of the quencher on the fluorophore and allowing fluorescence to be emitted. Higher fluorescence intensity can be considered indicative of higher responsiveness to cetuximab.

To achieve another objective, the present invention provides a method for providing information to predict responsiveness to cetuximab or the therapeutic efficacy of cetuximab, comprising the steps of treating a sample isolated from a cancer patient with the present composition and measuring the fluorescence intensity.

In a preferred embodiment of the present invention, when fluorescence intensity is detected, the subject can be determined to be responsive to cetuximab or to experience therapeutic efficacy from cetuximab.

To achieve another objective, the present invention provides a composition for diagnosing EGFR-positive cancer, comprising a complex, wherein the complex comprises: a peptide represented by the amino acid sequence of SEQ ID NO: 1, which is cleaved by a lysosomal enzyme present in a cancer cell; a fluorophore and a quencher bound to the peptide; and cetuximab bound to the C-terminus of the peptide.

In addition, the present invention provides a method for screening an anticancer drug having EGFR degradation activity, comprising the steps of: (i) treating an EGFR-positive cancer cell line with a candidate substance, wherein the cell line has been pretreated with a complex, and wherein the complex comprises: a peptide represented by the amino acid sequence of SEQ ID NO: 1, which is cleaved by a lysosomal enzyme present in a cancer cell; a fluorophore and a quencher bound to the peptide; and cetuximab bound to the C-terminus of the peptide; and (ii) measuring the fluorescence intensity generated by the complex.

### [ADVANTAGEOUS EFFECTS]

The cetuximab-conjugated probe (Cetux-probe) of the present invention operates via the same mechanism as cetuximab therapy, enabling rapid and accurate prediction of cetuximab's therapeutic efficacy and responsiveness. Furthermore, it was confirmed that the predictive capability of the activated fluorescence of the Cetux-probe is significantly higher than that of EGFR expression or KRAS mutation status. Therefore, the Cetux-probe of the present invention can be effectively utilized to predict responsiveness to cetuximab therapy by evaluating EGFR degradation.

### [BRIEF DESCRIPTION OF THE DRAWINGS]

Fig. 1 is a schematic diagram depicting the mechanism of action of the novel fluorescent probe, Cetux-probe, for visualizing target protein (EGFR) degradation, along with the binding mechanism of the Cetux-probe and its potential applications in precision medicine. It shows schematic representations of (a) cetuximab and (b) Cetux-probe, which share the same mechanism of action. The schematic illustrates EGFR-mediated internalization and lysosomal degradation. (c) Cetux-probe was synthesized by coupling C-probe to cetuximab via a 1-ethyl-3-(3-dimethylaminopropyl)carbodiimide (EDC)/N-hydroxysulfosuccinimide (sulfo-NHS) reaction. C-probe is an activatable peptide-based fluorescent probe activated by lysosomal enzymes. Furthermore, (d) Cetux-probe can be utilized to predict patient-specific responses to anti-EGFR therapy and to visualize targeted protein degradation.

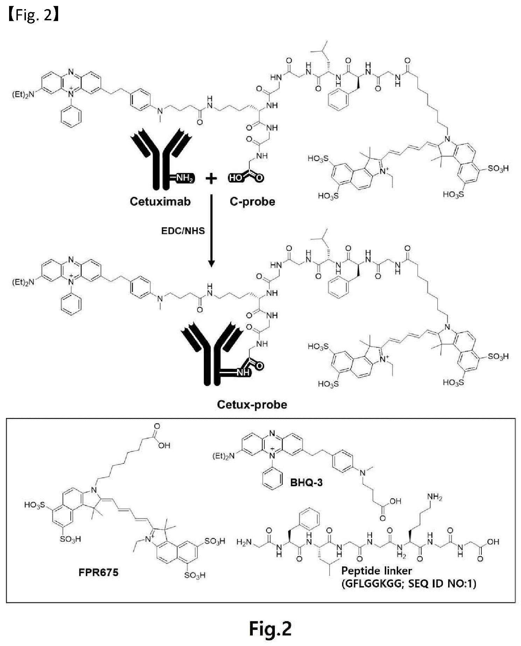

Fig. 2 is a schematic diagram illustrating the synthesis process of the Cetux-probe, wherein the conjugate is synthesized by amide coupling between the carboxylic acid group of the C-probe and the free amine group of cetuximab.

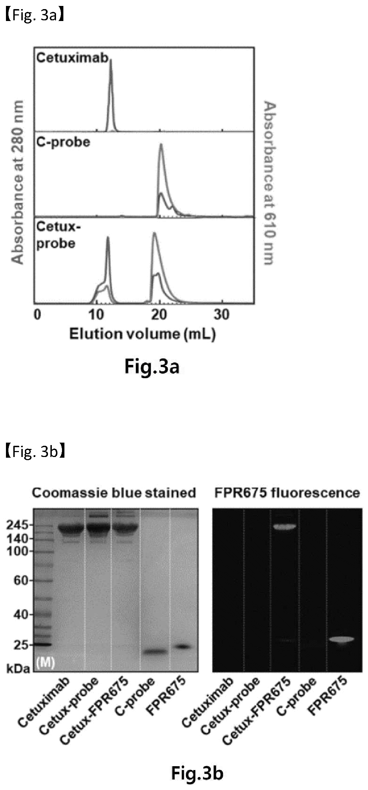

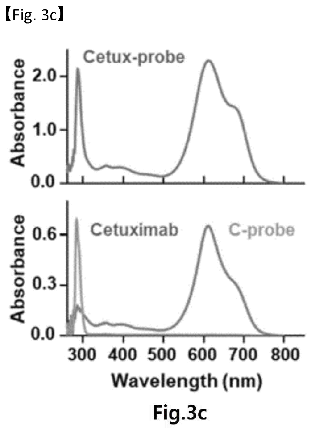

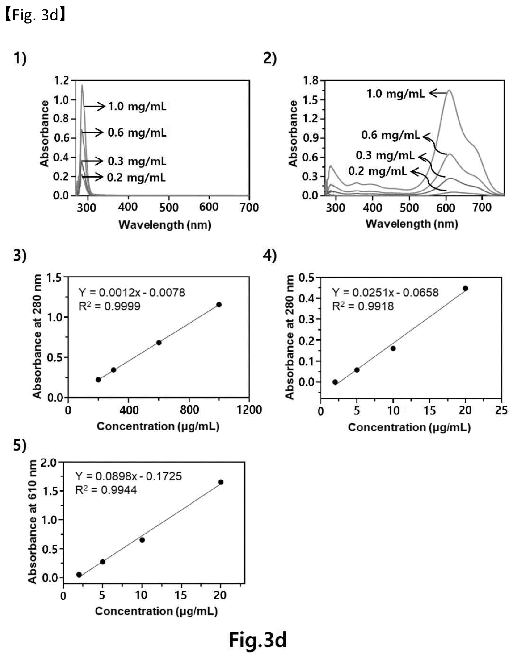

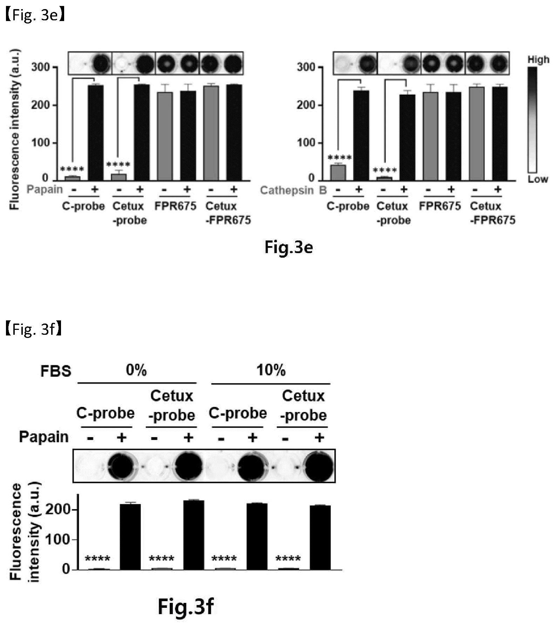

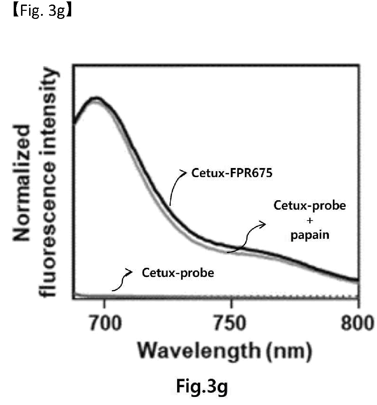

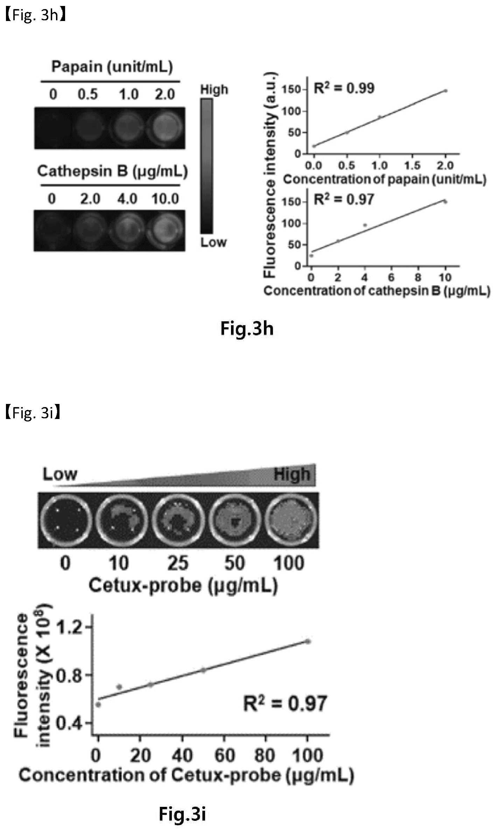

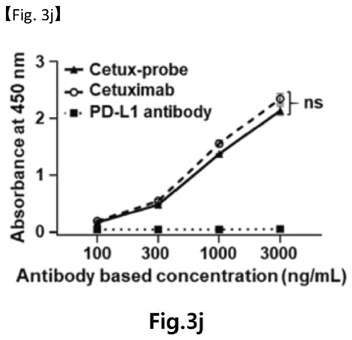

Fig. 3 shows data confirming the characterization of the Cetux-probe. Fig. 3a shows SEC-FPLC chromatograms of each of the crude reaction mixture, cetuximab, and C-probe, monitored at 280 nm (blue line) and 610 nm (red line). Fig. 3b shows the results of non-reducing SDS-PAGE analysis of cetuximab, Cetux-probe, Cetux-FPR675, C-probe, and FPR675, stained with InstantBlue^{™} Coomassie (left) and visualized in fluorescence mode (right). M indicates the molecular weight marker. Fig. 3c shows the UV-Vis absorption spectra of Cetux-probe (7.5 µM; top), cetuximab (0.6 mg/mL; blue line, bottom), and C-probe (10.0 µg/mL; orange line, bottom) in PBS. Fig. 3d shows the UV-Vis absorption spectra of 1) cetuximab and 2) C-probe in PBS, 3) an absorbance plot at 280 nm versus cetuximab concentration in PBS, 4) an absorbance plot at 280 nm versus C-probe concentration in PBS, and 5) an absorbance plot at 610 nm versus C-probe concentration in PBS. Fig. 3e shows data quantifying the fluorescence activation of C-probe, Cetux-probe, FPR675, and Cetux-FPR675 upon treatment with lysosomal enzymes papain (top) and cathepsin B (bottom). Fig. 3f shows data confirming the serum stability of C-probe and Cetux-probe. C-probe and Cetux-probe were each incubated in 10% FBS at 37°C for 24 hours. Fig. 3g shows the normalized fluorescence spectra of papain (5 units/mL)-activated Cetux-probe (1.5 µM, red curve) and Cetux-FPR675 (1.5 µM, black curve). Cetux-probe did not emit fluorescence without papain treatment (blue curve). Fig. 3h shows microplate fluorescence images (top) and quantitative analysis data (bottom) of Cetux-probe in the presence of papain (0~2.0 units/mL) and cathepsin B (0~10.0 µg/mL). Fig. 3i shows the fluorescence image (top) of a microplate containing lysates from CCK-81 cells treated with various concentrations of Cetux-probe (0~100 µg/mL) and the data from quantitative analysis (bottom) comparing Cetux-probe concentration and fluorescence intensity. Fig. 3j shows data confirming the binding affinity of Cetux-probe, cetuximab, and anti-PD-L1 antibody to EGFR, analyzed by ELISA.

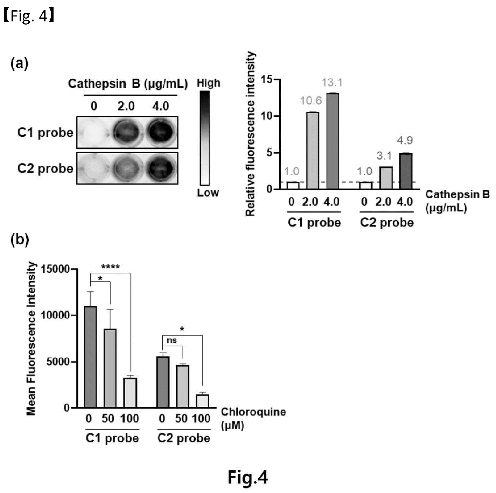

Fig. 4 shows data confirming the sensitivity of (a) fluorescence activity toward a lysosomal proteolytic enzyme (cathepsin B) and (b) fluorescence inhibition effect by a cellular lysosomal proteolysis inhibitor (chloroquine) using Cetux-probes manufactured based on two different peptide sequences cleaved by lysosomal enzymes.

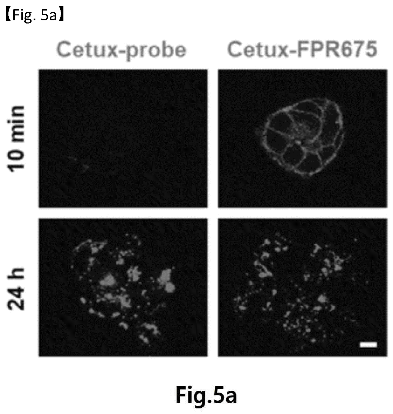

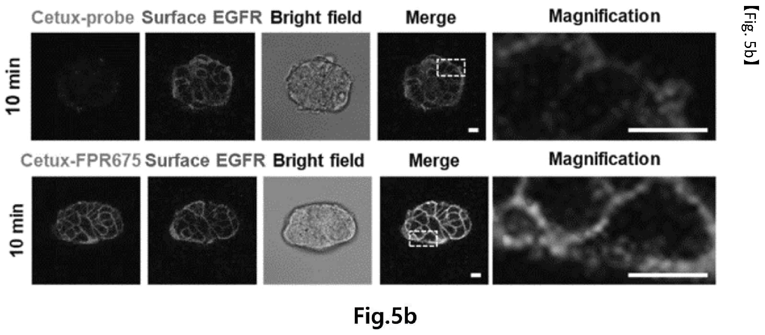

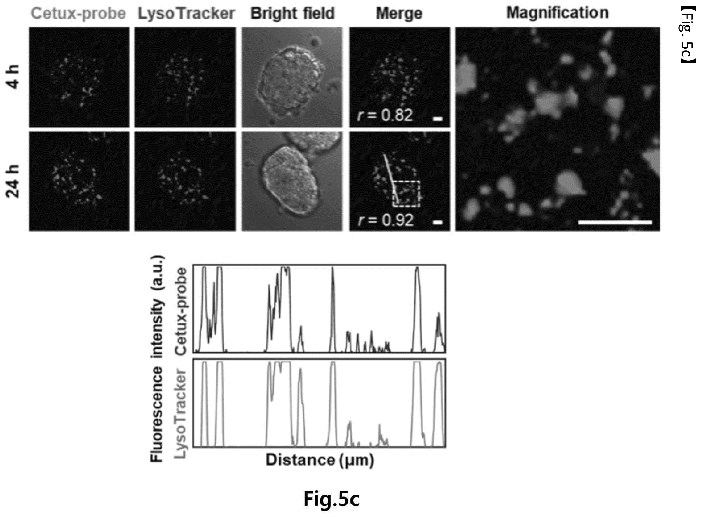

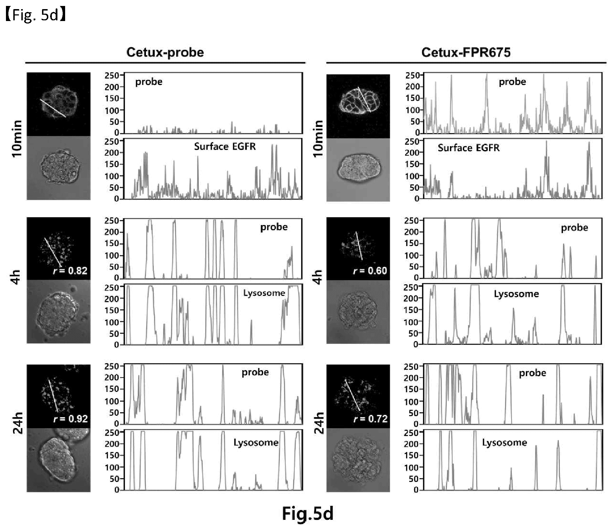

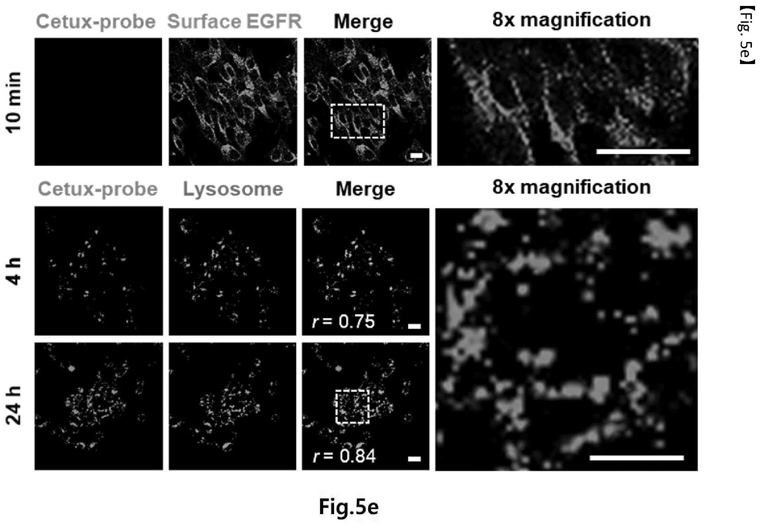

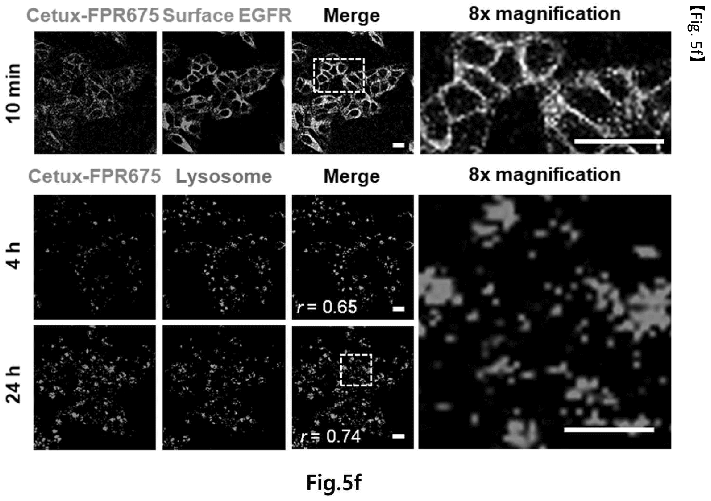

Fig. 5 shows data confirming the fluorescence activation of the Cetux-probe in CCK-81 cells. Fig. 5a shows confocal microscopy images of CCK-81 cells treated with the activatable Cetux-probe (1 µM, red) and the 'always-on' Cetux-FPR675 (1 µM, red) at 10 minutes and 24 hours post-probe treatment. Cetux-probe showed almost no detectable fluorescence at 10 min, but strong fluorescence was observed at 24 h. Cetux-FPR675 exhibited strong fluorescence at both 10 min and 24 h (scale bar: 20 µm). Fig. 5b shows fluorescence confocal microscopy images of CCK-81 cells 10 minutes after probe treatment. Cetux-probe (red) emitted a faint signal barely detectable at the cell membrane of CCK-81 cells, while Cetux-FPR675 (red) showed strong fluorescence that predominantly colocalized with surface EGFR (blue) distributed at the cell membrane under confocal microscopy (scale bar: 20 µm. The white dashed square indicates an enlarged area with a scale bar of 160 µm). Fig. 5c shows fluorescence confocal microscopy images (top) of CCK-81 cells treated with Cetux-probe (red) and LysoTracker (green) at 4 and 24 hours post-treatment, and data (bottom) profiling the fluorescence intensities of Cetux-probe and LysoTracker along the white line in the merged confocal image (scale bar: 20 µm. The white dashed square indicates an enlarged region at a scale bar of 160 µm). Fig. 5d shows fluorescence intensity profile data over time for Cetux-probe and Cetux-FPR675, respectively. Fluorescence intensities were obtained through simultaneous confocal imaging of surface EGFR (blue) or lysosomal markers (green) and the probe (red) in CCK-81 cells. Fig. 5e shows data confirming the intracellular localization of Cetux-probe (red) over time in KRAS-mutant colorectal cancer cells. Fig. 5f shows data confirming the intracellular localization of Cetux-FPR675 (red) over time in KRAS-mutant colorectal cancer cells. In Figs. 5e and 5f, the fluorescence localization of the probe (red) was analyzed using simultaneous confocal imaging with surface EGFR (blue) and lysosomal (green) markers. The degree of colocalization was quantified using the Pearson correlation coefficient (r). (White dashed squares indicate enlarged regions).

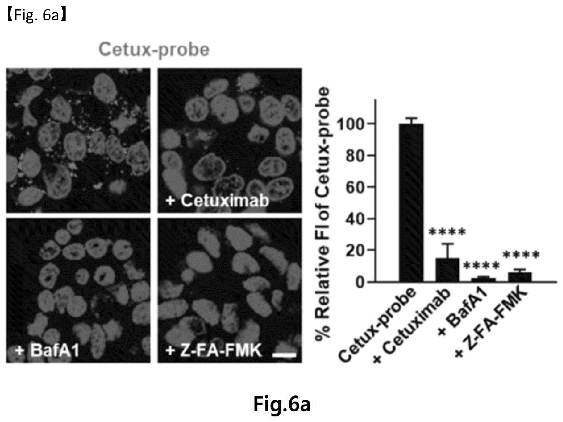

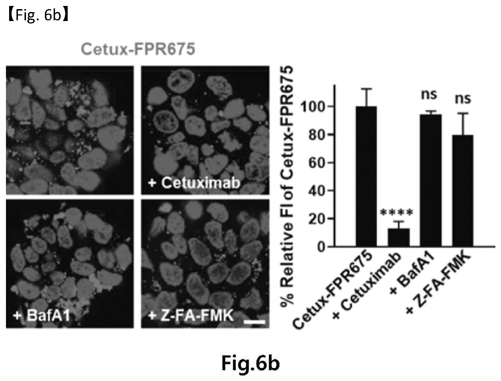

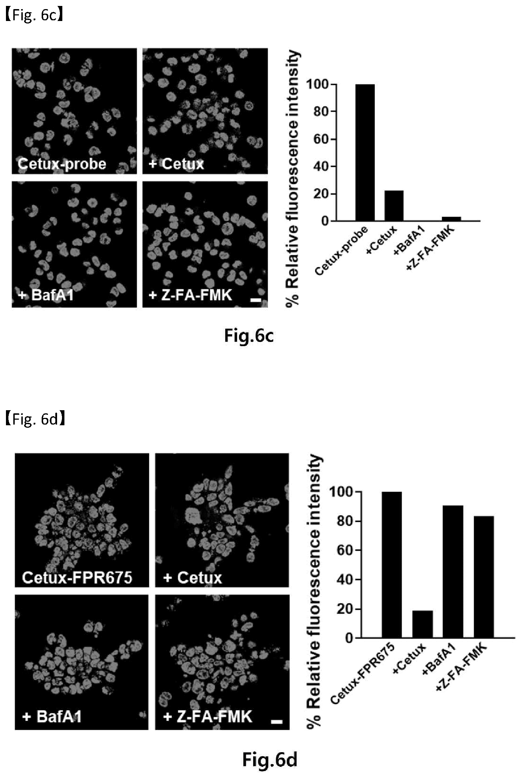

Fig. 6 shows data confirming Cetux-probe activation following inhibitor treatment. Fig. 6a and Fig. 6b show confocal microscopy images (top) and quantitative analysis data (bottom) of CCK-81 cells treated with Cetux-probe (1 µM, 4 hours) and Cetux-FPR675 (1 µM, 4 hours). FI denotes fluorescence intensity (scale bar: 40µm). Fig. 6c and Fig. 6d show confocal microscopy images (top) and quantitative analysis data (bottom) of HCT-8 cells treated with Cetux-probe (1 µM, 4 hours) and Cetux-FPR675 (1 µM, 4 hours). FI denotes fluorescence intensity in the Figs. (scale bar: 20 µm).

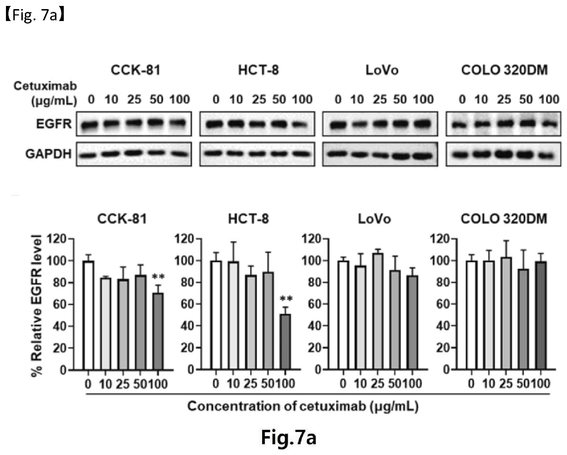

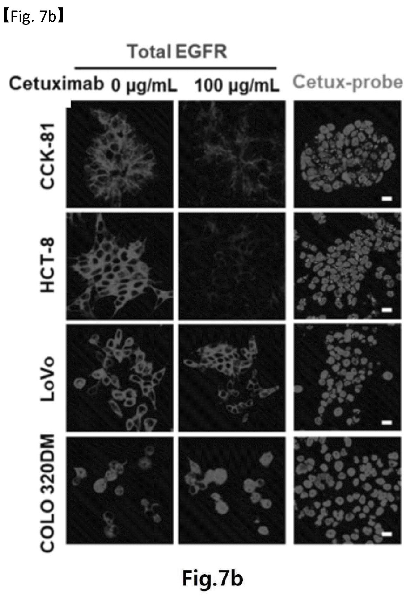

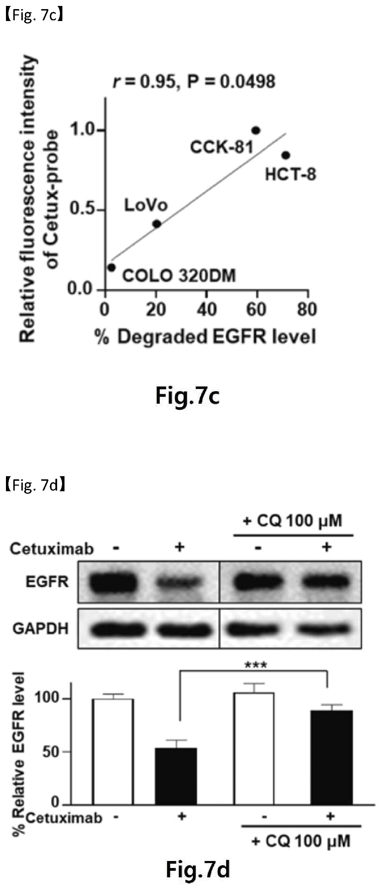

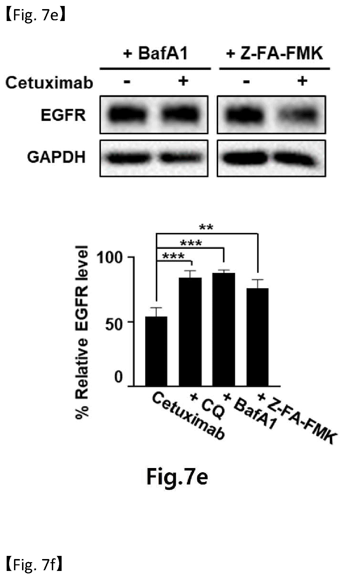

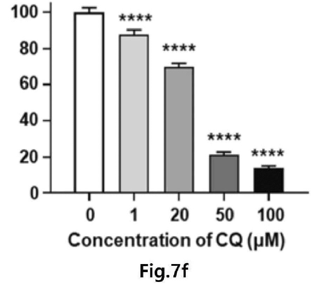

Fig. 7 shows data monitoring EGFR degradation in colorectal cancer cells using the Cetux-probe. Fig. 7a shows Western blot data quantifying total EGFR levels in CCK-81, HCT-8, LoVo, and COLO 320DM cells following cetuximab treatment (0~100 µg/mL), along with data quantifying relative EGFR levels. Relative EGFR levels were expressed as a percentage relative to the EGFR/GAPDH value in the cetuximab 0 µg/mL group, which was set as 100% (n = 3). Fig. 7b shows the total EGFR expression (green) in CCK-81, HCT-8, LoVo, and COLO 320DM cells treated with cetuximab (100 µg/mL, 24 hours), and fluorescence confocal microscopy images for the Cetux-probe (red, 1 µM, 24 hours) (scale bar: 20 µm). Fig. 7c shows data confirming the correlation between degraded EGFR (%) and relative fluorescence intensity of the Cetux-probe, with the plot evaluated using Pearson's correlation coefficient (r = 0.95, P = 0.0498). Fig. 7d shows Western blot analysis of EGFR levels in HCT-8 cells treated with cetuximab (100 µg/mL) for 24 hours in the presence of the inhibitor CQ (100 µM) (top), and the quantified data (bottom). Fig. 7e shows Western blot analysis of EGFR levels in HCT-8 cells treated with cetuximab (100 µg/mL) for 24 hours in the presence of the inhibitors BafA1 (100 nM) and Z-FA-FMK (100 µM) (top), and the quantified data (bottom). Fig. 7f shows data confirming the fluorescence intensity of the Cetux-probe (1 µM) in HCT-8 cells pretreated with various concentrations (0~100 µM) of CQ. Fluorescence intensity was measured as mean fluorescence intensity (MFI) using a flow cytometer.

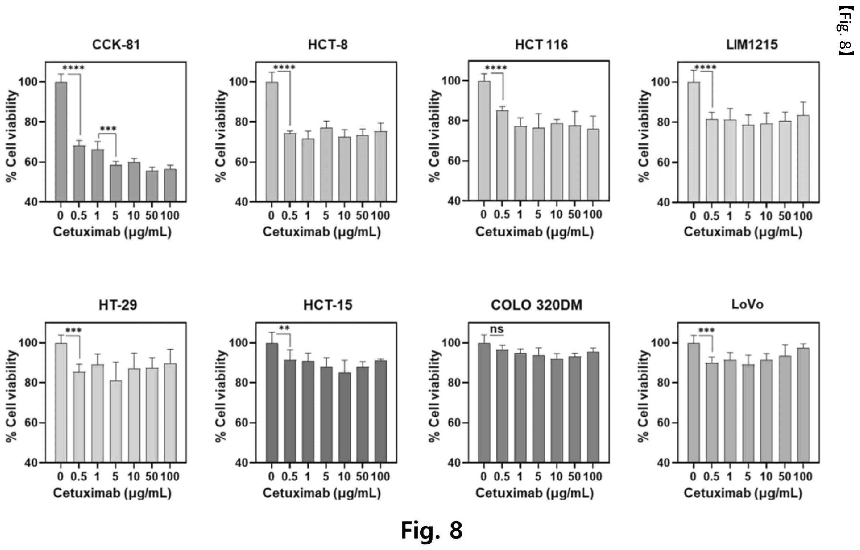

Fig. 8 shows data confirming the cytotoxicity of cetuximab in eight colorectal cancer cell lines. Cell survival rates were measured after treating KRAS wild-type (CCK-81, HT-29, LIM-1215, COLO 320DM) and KRAS mutant (HCT-8, HCT-116, HCT-15, LoVo) cells with various concentrations of cetuximab (0~100 µg/mL).

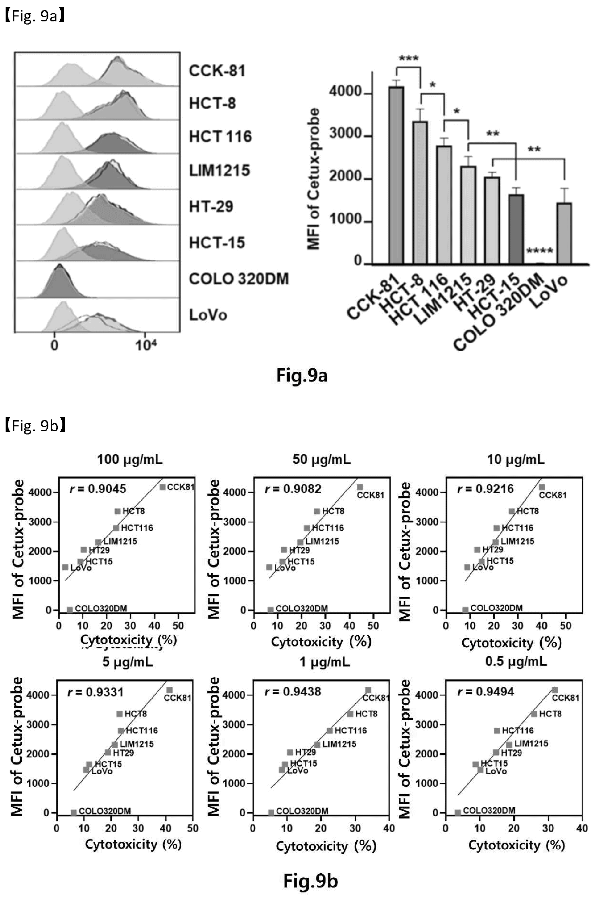

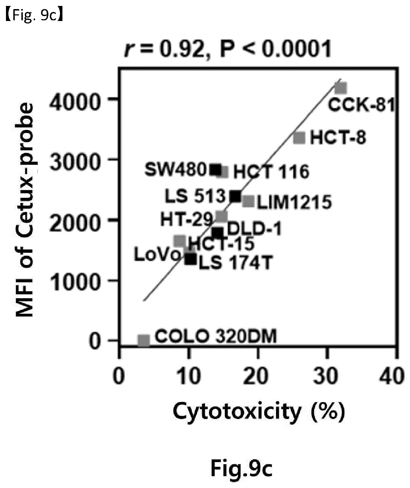

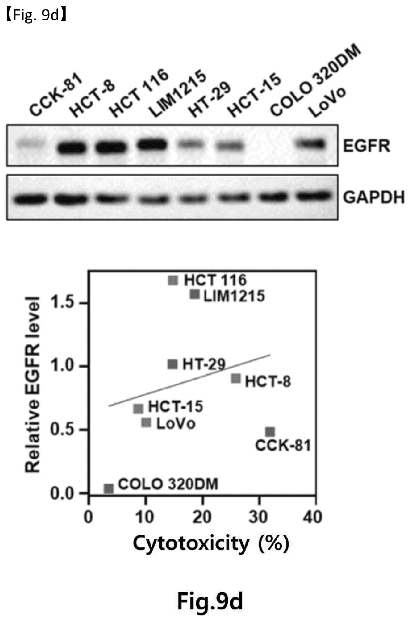

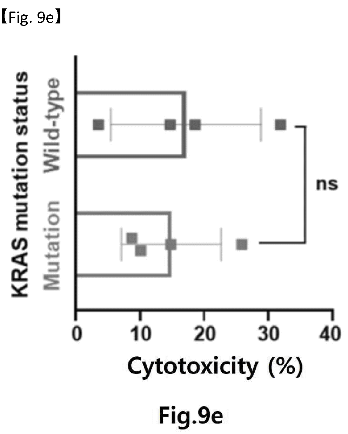

Fig. 9 shows data analyzing the correlation between cetuximab cytotoxicity and Cetux-probe fluorescence intensity in various colorectal cancer cell lines. Fig. 9a shows the flow cytometry histogram (left) and quantitative analysis data for mean fluorescence intensity (MFI) (right) of eight colorectal cancer cell lines treated with 10 nM Cetux-probe for 24 hours. Fig. 9b shows data analyzing the correlation between the cytotoxicity of cetuximab and the mean fluorescence intensity of the Cetux-probe when treating eight colorectal cancer cell lines with various concentrations of cetuximab (100 µg/mL, 50 µg/mL, 10 µg/mL, 5 µg/mL, 1 µg/mL, 0.5 µg/mL) for 48 hours. Fig. 9c shows data analyzing the correlation between cetuximab cytotoxicity and the mean fluorescence intensity of the Cetux-probe when treating 12 colorectal cancer cell lines with cetuximab (0.5 µg/mL). Fig. 9d shows the EGFR levels confirmed by Western blot in 8 colorectal cancer cell lines (top) and the correlation plot between the relative EGFR levels and the cytotoxicity of cetuximab (0.5 µg/mL) (bottom). The correlation was assessed using the Pearson correlation coefficient (r = 0.24). Fig. 9e shows data confirming the cytotoxicity of cetuximab (0.5 µg/mL) in KRAS wild-type and KRAS mutant cells. Red dots in the plot represent KRAS mutant cells, while blue dots represent KRAS wild-type cells.

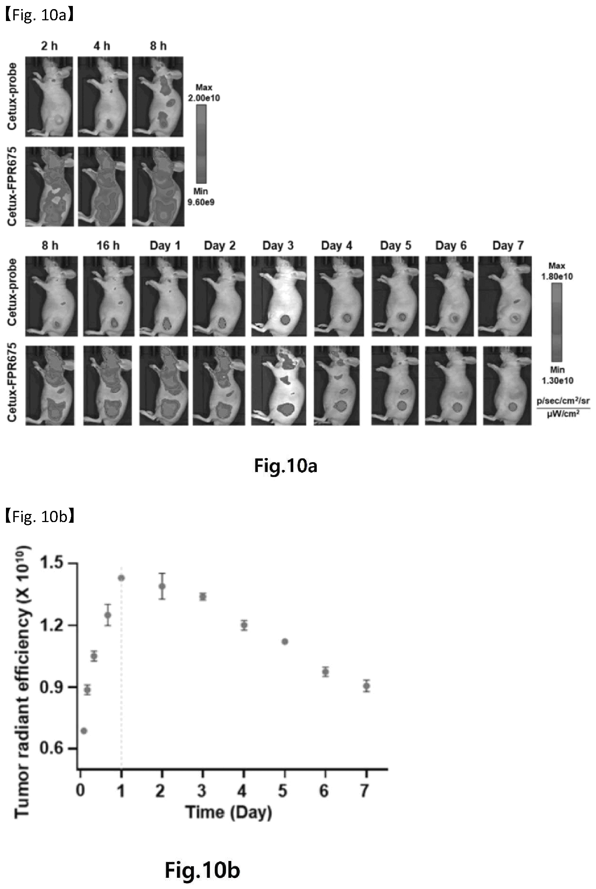

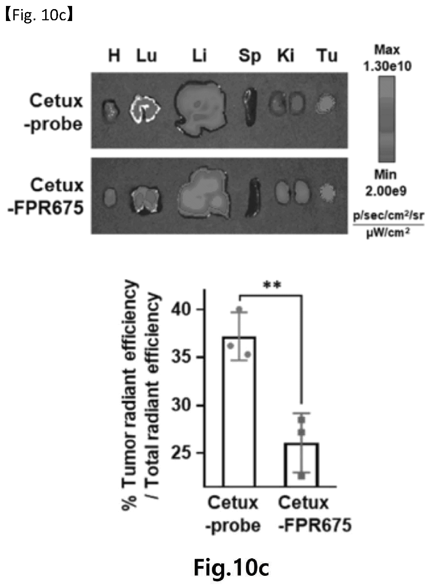

Fig. 10 shows data observing the in vivo distribution and tumor-enhanced fluorescence of the Cetux-probe in a colorectal cancer cell-derived xenograft mouse model. Fig. 10a shows IVIS fluorescence imaging images of Cetux-probe and Cetux-FPR675 in mice bearing HT-29 tumors over 7 days post-probe injection. Fig. 10b shows data analyzing the average radiant efficiency in the tumor region of mice injected with the Cetux-probe, with fluorescence imaging at 2, 4, and 8 hours separately normalized. Fig. 10c shows ex vivo fluorescence imaging images of excised major organs and tumors (top) from mice with HT-29 tumors, and data quantifying the fluorescence radiant efficiency for the tumor as the ratio of tumor radiant efficiency to total radiant efficiency in ex vivo imaging using Living Image software (n = 3) (bottom) (H: heart, Lu: lung, Li: liver, Sp: spleen, Ki: kidney, Tu: tumor).

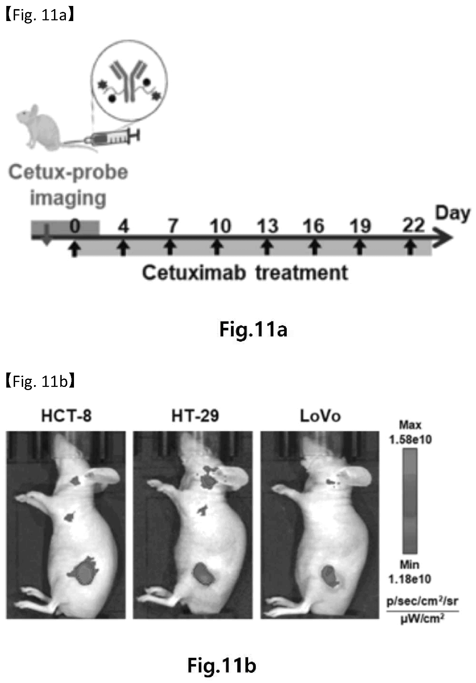

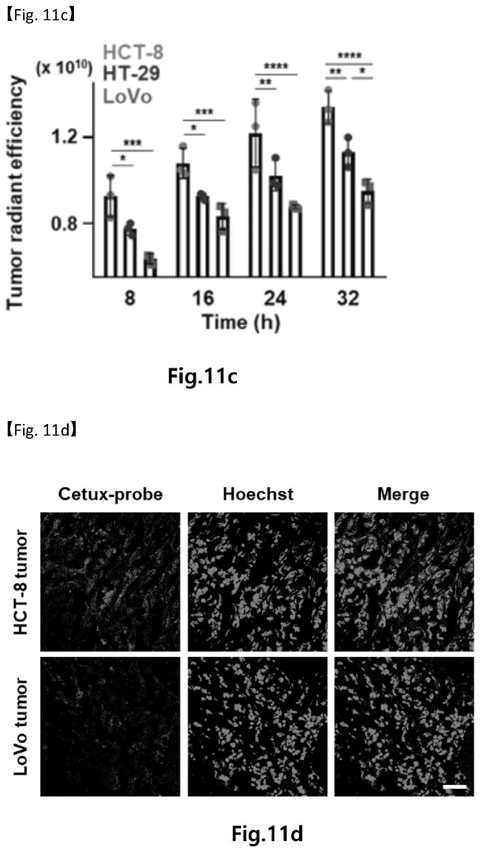

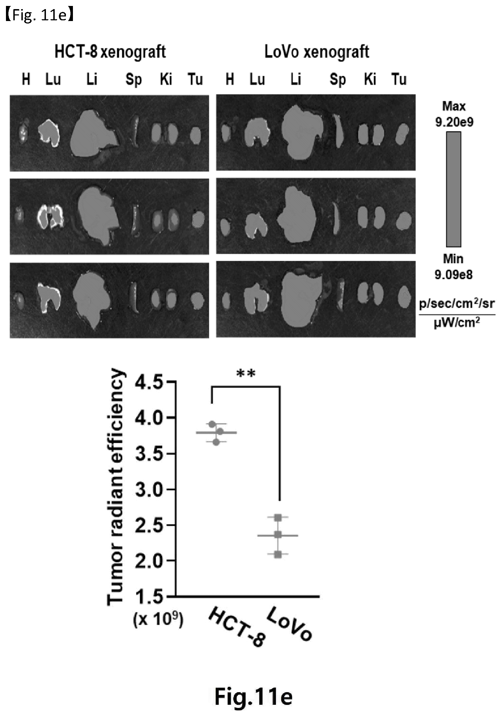

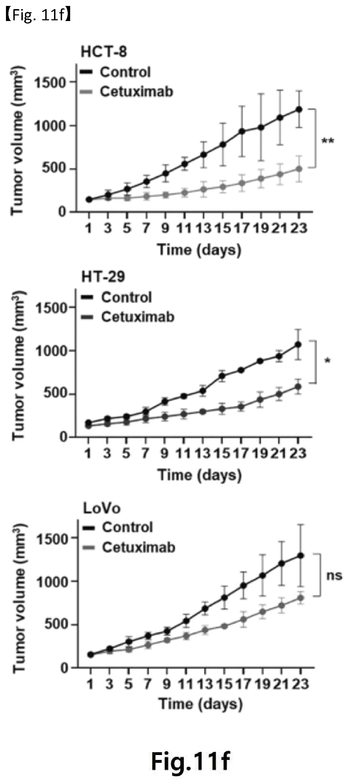

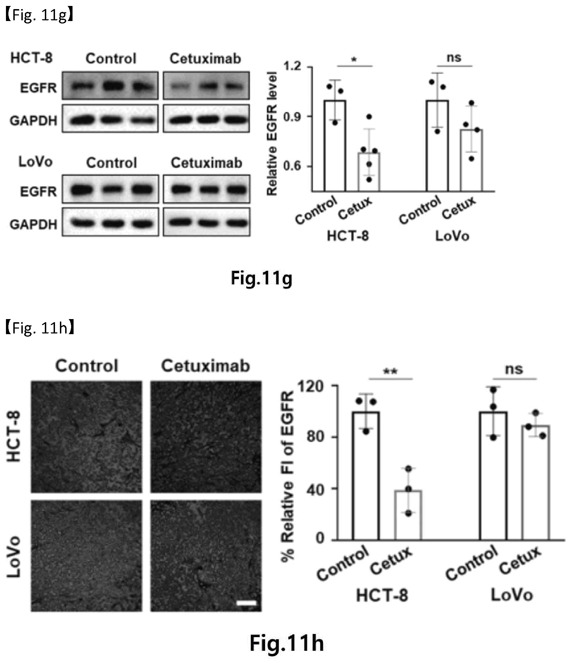

Fig. 11 shows data predicting therapeutic responsiveness to cetuximab treatment by measuring EGFR degradation in vivo using the Cetux-probe. Fig. 11a is a schematic diagram showing the in vivo experimental timeline of Cetux-probe imaging and cetuximab treatment in animal experiments. Fig. 11b shows IVIS fluorescence imaging of mice bearing HCT-8 tumors, HT-29 tumors, and LoVo tumors 32 hours after intravenous administration of Cetux-probe (10 nmol). The tumor sizes in HCT-8, HT-29, and LoVo xenograft mice were nearly identical when imaged prior to initiating cetuximab treatment. Fig. 11c shows data quantifying the average radiant efficiency of the Cetux-probe in the tumor regions in vivo at 8, 16, 24, and 32 hours after Cetux-probe administration. Fig. 11d shows fluorescence images of tumor tissue sections from HCT-8 and LoVo mice treated with Cetux-probe (red) (scale bar: 50µm). Fig. 11e shows ex vivo fluorescence imaging data of excised major organs and tumors from mice bearing HCT-8 and LoVo tumors 32 hours after intravenous administration of Cetux-probe, along with quantitative analysis results for the average radiant efficiency of Cetux-probe in the indicated tumors (H: heart, Lu: lung, Li: liver, Sp: spleen, Ki: kidney, Tu: tumor). Fig. 11f shows data observing tumor growth in mice bearing HCT-8 tumors, HT-29 tumors, and LoVo tumors. Control mice were injected with PBS (control group n = 3, cetuximab-treated group n = 5). Fig. 11g shows Western blot images of EGFR and GAPDH expression in tumor lysates (top) and quantitative EGFR/GAPDH levels (bottom). Fig. 11h shows immunohistochemical staining for EGFR in tumor tissue collected from mice bearing HCT-8 tumors and LoVo tumors treated with cetuximab (left) and quantitative analysis data for EGFR levels (right). The relative fluorescence intensity of EGFR in the control tumor tissue was set to 100%.

### [MODE FOR THE INVENTION]

The present invention is described in detail below.

The present invention provides a composition for predicting cetuximab responsiveness or confirming the prognosis regarding cetuximab treatment efficacy, comprising a complex, wherein the complex comprises: a peptide represented by the amino acid sequence of SEQ ID NO: 1, which is cleaved by a lysosomal enzyme present in a cancer cell; a fluorophore and a quencher bound to the peptide; and cetuximab bound to the C-terminus of the peptide.

In the present invention, the fluorophore may be bound to the N-terminus of the peptide, and the quencher may be bound to the epsilon amine group of a lysine or arginine amino acid residue of the peptide.

In the present invention, the fluorophore may be selected from the group consisting of fluorescein, FITC (fluorescein isothiocyanate), Oregon green, Texas red, Cy2, Cy3, Cy3B, Cy3.5, Cy5, Cy5.5, Cy7, indocarbocyanine, rhodamine, oxacarbocyanine, thiacarbocyanine, merocyanine, pyrodyloxazole, nitrobenzoxadiazole, benzoxadiazole, Nile red, Nile orange, acridine yellow, aumarine, crystal violet, and malachite green.

In the present invention, the quencher may be selected from the group consisting of TAMRA (6-carboxytetramethylrhodamine), BHQ1 (black hole quencher 1), BHQ2 (black hole quencher 2), BHQ3 (black hole quencher 3), NFQ (nonfluorescent quencher), dabcyl, Eclipse, DDQ (Deep Dark Quencher), Blackberry Quencher, and Iowa black.

In the present invention, the cancer may be an epidermal growth factor receptor (EGFR)-positive cancer, which may be colorectal cancer, head and neck cancer, or lung cancer known to be treated with cetuximab, and preferably may be EGFR-positive colorectal cancer.

In the present invention, the complex can penetrate cancer cells and be cleaved by enzymes present within the lysosomes of the cancer cells, thereby relieving the quenching effect of the quencher on the fluorophore and allowing the fluorophore to emit fluorescence. Higher fluorescence intensity indicates higher responsiveness to cetuximab.

The inventors of the present invention previously developed an EGF-Probe in prior research (Korean Patent Publication No. 10-2022-0021509). While the EGF-Probe and the Cetux-probe of the present invention share the same high specificity for binding to EGFR, EGF is known as a natural ligand for EGFR that promotes cancer initiation and growth. Therefore, the present invention aimed to develop a safer substance than the EGF-Probe.

Since cetuximab is also a targeted therapy that treats cancer by targeting EGFR and is expected to be suitable as a tool for predicting cancer treatment responsiveness, the present invention developed a predictive probe that operates via the same mechanism as the therapeutic agent. This probe aims to predict responsiveness to EGFR-targeted therapies like cetuximab. The mechanism by which the cetuximab therapeutic binds to EGFR, undergoes internalization, and is degraded by lysosomal enzymes offers the advantage that the Cetux-probe, once bound to cetuximab, can accurately mimic this mechanism better than the EGF-probe.

In a specific embodiment of the present invention, a cetuximab-conjugated probe (Cetux-probe) capable of predicting drug responsiveness to cetuximab was prepared using the schematic diagram of Fig. 1 and the synthesis method of Fig. 2. It was confirmed that the Cetux-probe activates fluorescence proportionally to the concentration of lysosomal enzymes and the amount of probe used. Furthermore, its binding affinity for human recombinant EGFR protein was found to be nearly identical to that of cetuximab.

In another specific embodiment of the invention, treatment of colorectal cancer cell lines with the Cetux-probe confirmed that the internalized Cetux-probe was activated by lysosomal degradation. Furthermore, treatment of various colorectal cancer cell lines with the Cetux-probe revealed a strong correlation between cetuximab-induced cytotoxicity and the fluorescence intensity of the Cetux-probe.

Furthermore, in animal models, the drug responsiveness to cetuximab using the Cetux-probe was confirmed, demonstrating that the fluorescence intensity of the Cetux-probe correlated with the degree of EGFR degradation and the therapeutic efficacy of cetuximab in mice with colorectal cancer.

From another perspective, the present invention relates to a method for providing information to predict responsiveness to cetuximab or the therapeutic efficacy of cetuximab, comprising the steps of treating a sample isolated from a cancer patient with the composition and measuring the fluorescence intensity.

In the present invention, when fluorescence intensity is detected, the subject can be determined to be responsive to cetuximab or to experience therapeutic efficacy from cetuximab.

From another perspective, the present invention relates to a composition for diagnosing EGFR-positive cancer, comprising a complex, wherein the complex comprises: a peptide represented by the amino acid sequence of SEQ ID NO: 1, which is cleaved by a lysosomal enzyme present in a cancer cell; a fluorophore and a quencher bound to the peptide; and cetuximab bound to the C-terminus of the peptide.

In the present invention, it was confirmed that the Cetux-probe internalized into cells via EGFR is activated by lysosomal degradation, resulting in increased fluorescence intensity. Therefore, it can be utilized as a diagnostic composition for EGFR-positive cancer, preferably EGFR-positive colorectal cancer.

From another perspective, the present invention relates to a method for screening an anticancer drug having EGFR degradation activity, comprising the steps of: (i) treating an EGFR-positive cancer cell line with a candidate substance, wherein the cell line has been pretreated with a complex, and wherein the complex comprises: a peptide represented by the amino acid sequence of SEQ ID NO: 1, which is cleaved by a lysosomal enzyme present in a cancer cell; a fluorophore and a quencher bound to the peptide; and cetuximab bound to the C-terminus of the peptide; and (ii) measuring the fluorescence intensity generated by the complex.

In the present invention, when the fluorescence intensity is detected, the candidate substance can be selected as having EGFR degradation activity.

In the present invention, it was confirmed that there is a significant and strong correlation between cetuximab-induced EGFR degradation and the fluorescence activation of the Cetux-probe. Therefore, it can be utilized for screening candidate substances having EGFR degradation activity equal or superior to that of cetuximab.

The present invention will now be described in more detail through the following examples.

These examples are provided solely for illustrative purposes and should not be construed as limiting the scope of the invention. This will be apparent to those skilled in the art.

### Example 1: Preparation of Cetux-probe

### 1-1: Preparation of Cetux-probe

In the present invention, a Cetux-probe capable of predicting drug responsiveness to cetuximab was manufactured. As shown in Fig. 1, the present invention developed the Cetux-probe by conjugating a lysosomal enzyme-activated peptide-based probe (C-probe) to cetuximab.

The C-probe was synthesized using a previously disclosed method. The C-probe comprises a GFLG substrate peptide reported to exhibit lysosomal enzyme-specific cleavage, conjugated with the near-infrared fluorophore Flamma^{™} Fluors 675 (FPR675) and the dark quencher (BHQ-3).

Specifically, as shown in Fig. 2, the peptide (GFLGGKGG; SEQ ID NO: 1) and FPR675 NHS ester were reacted with dimethylaminopyridine and N-methylmorpholine, and the peptide-FPR675 conjugate was reacted with BHQ-3 NHS ester to generate the C-probe. The product was purified by RP-HPLC on a C18 column.

Next, the C-probe was bound to cetuximab via an EDC/N-hydroxysulfosuccinimide (sulfo-NHS) reaction to produce the Cetux-probe. C-probe (5.12 mg, 2.57 µmol), sulfo-NHS (6.98 mg, 32.16 µmol), and EDC (1-ethyl-3-(3-dimethylaminopropyl)carbodiimide, 2.47 mg, 12.86 µmol) were shaken for 15 minutes in phosphate-buffered saline (PBS; pH 8.0), followed by the addition of 1.8 mL of cetuximab (21 mg/mL). The mixture was reacted at 25°C for 2 hours and purified using an AKTA Purifier 100 FPLC system (Cytiva, USA) equipped with a Superdex 200 Increase 10/300 GL column (#28-9909-44, Cytiva). The purified Cetux-probe was stored in PBS (pH 7.4) at 4°C for subsequent use.

As a control, Cetux-FPR675 was synthesized by conjugating only the fluorophore (FPR675) to cetuximab. Cetux-FPR675 was synthesized by conjugating FPR675 NHS ester (1.07 mg, 1.03 µmol) with 1.8 mL of cetuximab (21 mg/mL) using the same method as for the Cetux-probe conjugate.

### 1-2: Characterization of Cetux-probe

To characterize the synthesized Cetux-probe, SEC-FPLC (fast protein size-exclusion liquid chromatography) was performed and monitored by detecting specific absorbance wavelengths for the Cetux-probe and Cetux-FPR675 (cetuximab λmax 280 nm and C-probe λmax 610 nm).

Absorption and fluorescence spectra were recorded using a Cary 300 spectrophotometer (Agilent Technologies, USA) and a fluorescence spectrophotometer F-7000 (Hitachi-High-Tech, Japan), respectively. The fluorescence spectrum was obtained by scanning the emission fluorescence from 680 nm to 800 nm after excitation at 672 nm.

As shown in Fig. 3a, compared to free cetuximab, the conjugated cetuximab was eluted earlier from the reaction mixture at elution volumes of 9 to 11 mL. Simultaneous absorbance signals were observed at 280 nm and 610 nm, indicating that the C-probe was successfully bound to cetuximab.

Cetux-probe was purified by SEC-FPLC and confirmed by non-reducing SDS-PAGE. First, cetuximab, Cetux-probe, and Cetux-FPR675 were quantified at the same concentration, then samples mixed with 4X non-reducing loading buffer were loaded onto an 8% (w/v) SDS-PAGE gel. FPR675 fluorescence in the gel was detected using an iBright^{™} FL1000 imaging system (Invitrogen, USA) in FPR675 fluorescence mode (excitation: 608-632 nm, emission: 675-720 nm). The gel was then stained with InstantBlue^{™} Coomassie dye, and the proteins in each sample were identified using the protein visible light mode of the same instrument.

As shown in Fig. 3b, when proteins were stained with InstantBlue^{™} Coomassie dye, the Cetux-probe exhibited a strong band only at the cetuximab position on the SDS-PAGE, with no band at the C-probe position, indicating that unbound C-probe was successfully removed (Fig. 3b, left lanes 2 and 4).

Furthermore, on the SDS-PAGE gel, the Cetux-probe did not emit fluorescence, whereas Cetux-FPR675, used here as a comparative formulation for the Cetux-probe, exhibited strong fluorescence (Fig. 3b, right lanes 2 and 3). This is consistent with the activatable properties of the Cetux-probe.

The average number of C-probes bound to cetuximab in the Cetux-probe was evaluated by measuring the absorbance of cetuximab and C-probe at various concentrations using a UV/Vis spectrophotometer. As shown in Fig. 3c and Fig. 3d, the absorbance of the Cetux-probe at 280 nm for cetuximab and at 610 nm for the C-probe was compared to standard curves generated for cetuximab and the C-probe. The results showed that 1.30 ± 0.11 C-probes were bound to each cetuximab. This indicates that the C-probe successfully attached to cetuximab to form the Cetux-probe.

### 1-3: Confirmation of Fluorescence Intensity Based on C-probe Sequence

In this invention, a new activation probe (C2 probe) was developed based on a peptide with a different sequence. The C2 probe was composed of a lysosomal enzyme-activated peptide conjugated with the near-infrared fluorophore Flamma^{™} Fluors 675 (FPR675) and the dark quencher (BHQ-3). The C2 probe differs from the C-probe prepared in <Example 1-1> in that it is composed of a peptide with a core cleavage sequence based on GGFG (GGFGGKGG; SEQ ID NO: 2).

Specifically, to synthesize the peptide-FPR675 conjugate, one equivalent of the peptide and 1.2 equivalents of FPR675 NHS ester were reacted for 2 hours. After the reaction, the product was purified by RP-HPLC using a C18 column and then lyophilized. Subsequently, for BHQ-3 conjugation, the peptide-FPR675 conjugate was treated with 50% trifluoroacetic acid/dichloromethane for 30 minutes to expose the amine group. To this, 1 equivalent of the reactant, 1.2 equivalents of BHQ-3 NHS ester, and 10 equivalents of N,N-diisopropylethylamine were added and allowed to react for 1 hour. The final product was purified by RP-HPLC using a C18 column and lyophilized."

Next, the Cetux-probe was synthesized using the C2 probe as described in <Example 1-1>.

Using the Cetux-probes prepared based on the two different peptide sequences cleaved by lysosomal enzymes, the sensitivity to the fluorescent activity of the lysosomal proteolytic enzyme (cathepsin B) and the fluorescent inhibitory effect of the cellular lysosomal proteolysis inhibitor (chloroquine) were confirmed. In Fig. 5, C1 probe denotes the Cetux-probe prepared using the C-probe, and C2 probe denotes the Cetux-probe prepared using the C2 probe (Cetux-C2 probe).

As shown in Fig. 5a, both probes exhibited fluorescence quenching in the absence of cathepsin B. However, as the cathepsin B concentration increased, fluorescence signals appeared. At this point, the C1 probe responded more sensitively to cathepsin B than the C2 probe, resulting in a stronger activation of the fluorescence signal.

Furthermore, as shown in Fig. 5b, both probes exhibited activated fluorescence intensity in EGFR-expressing colorectal cancer cells. In cells pretreated with chloroquine, the fluorescence intensity of each probe decreased as the chloroquine concentration increased. At this time, the C1 probe exhibited stronger intracellular fluorescence signal activity than the C2 probe and responded sensitively to chloroquine, showing a more pronounced inhibition of fluorescence intensity.

The C1 probe and C2 probe exhibit highly similar fluorescence recovery characteristics for basic lysosomal enzymes. Furthermore, when the C1 probe and C2 probe were incorporated into the EGF-probe previously developed by the inventors of the present invention, their fluorescence recovery characteristics were also found to be highly similar.

In contrast, when the C1 probe and C2 probe were bound to cetuximab, the fluorescence recovery capability of the Cetux-probe (using C1) was confirmed to be significantly superior to that of the Cetux-C2 probe. Therefore, in this invention, subsequent experiments were performed using the Cetux-probe, which possesses excellent fluorescence recovery capability.

### Example 2: Confirmation of Cetux-probe Activation

### 2-1: Confirmation of Cetux-probe Activation by Lysosomal Enzymes

Since the Cetux-probe was designed to be activated by lysosomal enzymes, its activation by these enzymes was confirmed.

Cetux-probe, C-probe, Cetux-FPR675, and FPR675 were adjusted to the same concentration (1 µM FPR675 equivalent) and dispensed into a 96-well microplate. Each sample was incubated with PBS buffer (pH 7.4) containing papain (0-2.0 units/mL) or PBS buffer (pH 7.4) containing cathepsin B (0-10.0 µg/mL) with 2-(N-morpholino)ethanesulfonic acid (MES) at 37°C for 1 hour. Fluorescence intensity in each well was then measured using the iBright^{™} FPR675 fluorescence mode. Fluorescence intensity was quantified using Image J software (National Institutes of Health, USA), and the coefficient of determination (R²) was determined using Prism GraphPad software (MA, USA).

The results showed that in the absence of enzymes, FPR675 fluorescence was significantly inhibited by BHQ-3, and neither the C-probe nor the Cetux-probe exhibited fluorescence. Upon application of papain and cathepsin B, the fluorescence of the Cetux-probe dramatically increased by 13.5±0.2-fold and 24.0±4.8-fold, respectively. In contrast, unlike the activatable Cetux-probe, the "always-on" probe Cetux-FPR675 exhibited strong fluorescence regardless of enzyme presence (Fig. 3e).

Fluorescence in the uncleaved probe is quenched by fluorescence resonance energy transfer (FRET) between the fluorophore and the quencher. Consistent with this, both the C-probe and Cetux-probe remained quenched even after incubation at 37°C for 24 hours with 10% FBS (Fig. 3f).

The fluorescence emission spectrum of the activated Cetux-probe was identical to that of Cetux-FPR675, indicating that FPR675 was unchanged by activation (Fig. 3g).

Furthermore, the fluorescence intensity of the Cetux-probe increased in a dosedependent manner when exposed to different concentrations of papain (R² = 0.99) or cathepsin B (R² = 0.97), or when treated with various concentrations of the Cetux-probe itself (R² = 0.97) (Fig. 3h and Fig. 3i). These results indicate that the Cetux-probe activates fluorescence proportionally to the concentration of lysosomal enzymes and the amount of probe used.

### 2-2: Evaluation of Cetux-probe

Affinity for EGFR To determine whether conjugation of the C-probe to cetuximab could impair cetuximab's EGFR binding ability, the affinity of cetuximab for EGFR was evaluated. An enzyme-linked immunosorbent assay (ELISA) against human recombinant EGFR protein was performed for various concentrations (100~3000 ng/mL) of cetuximab, Cetux-probe, and anti-PD-L1 antibody.

Binding affinity for human recombinant EGFR was evaluated using the Cetuximab (Human) ELISA Kit according to the manufacturer's protocol. Briefly, buffer for the assay was added to each well, followed by the addition of standard reagents (cetuximab), cetuximab, Cetux-probe, or PD-L1 antibody. Next, samples were incubated at room temperature (RT) for 30 minutes. The reaction solution was then removed, and wells were washed three times. HRP-conjugated probe solution was added, and incubation continued at RT for an additional 30 minutes. After repeated washing, TMB (3,3',5,5'-tetramethylbenzidine) chromogenic substrate solution was added and incubated for 10 minutes at room temperature under light-protected conditions. Finally, a stopping solution was added, and the absorbance of each well was detected at 450 nm using a VersaMax^{™} microplate reader (Molecular Devices Corp., USA). The concentrations of the standard reagents used were 0, 100, 300, 1000, and 3000 ng/mL of cetuximab moiety.

As a result, as shown in Fig. 3j, the binding affinity of the Cetux-probe for human recombinant EGFR protein was confirmed to be nearly identical to that of cetuximab, while the anti-PD-L1 used as a control showed almost no binding affinity for human recombinant EGFR protein. These results indicate that conjugation of cetuximab with the C-probe does not reduce the affinity of cetuximab for EGFR.

### Example 3: Confirmation of Cetux-probe Activation in KRAS Wild-type Colorectal Cancer Cells

Cetux-probe activation was confirmed in KRAS wild-type colorectal cancer cells (CCK-81 cells) following lysosomal degradation.

The CCK-81 cell line was purchased from the JCRB Cell Bank (Japanese Collection of Research Bioresources Cell Bank) and cultured in the recommended medium containing 10% FBS and 1% antibiotics (streptomycin and 100 units/mL penicillin) at 37°C under humidified conditions with 5% CO₂.

Cells were seeded at a density of 3.0 × 10⁵ cells/well in confocal dishes and treated with Cetux-probe and Cetux-FPR675 at a concentration of 1 µM each. To co-label lysosomes, cells were treated with 2 µM LysoTracker Green for 2 hours. Fluorescent images of the cells were acquired using a TCS SP8 confocal laser scanning microscope (Leica, Wetzlar, Germany). To quantify the degree of overlap between the two fluorescent signals, the Pearson correlation coefficient (r) for co-localization was measured. Fluorescence intensity profiles showed the distribution of each fluorescence along a line. Fluorescence intensity was calculated as the fluorescence intensity per cell nucleus in each image using Image J software and expressed as relative fluorescence intensity.

To co-label EGFR on the cell surface, the treated probes were washed, and the cells were incubated with rabbit anti-human EGFR antibody at 4°C for 10 minutes. After two washes with PBS (pH 7.4), Alexa Fluor 488-labeled goat anti-rabbit antibody was applied as the secondary antibody at 4°C for 30 minutes. To stain total EGFR on cells, cells were fixed and permeabilized using the Cytofix/Cytoperm^{™} kit, then blocked with 1% (w/v) BSA at room temperature for 30 minutes. Cells were then incubated with the same anti-EGFR antibody at room temperature for 30 minutes, washed with PBS, and stained with the same secondary antibody at room temperature for 30 minutes. Fluorescent images of stained cells were acquired using a confocal microscope and quantified using Image J software.

The results showed that KRAS wild-type colorectal cancer cells (CCK-81 cells) exhibited sparse fluorescence after 10 minutes of treatment with the Cetux-probe and strong fluorescence after 24 hours, while cells treated with Cetux-FPR675 showed strong fluorescence at both 10 minutes and 24 hours (Fig. 5a).

To monitor the intracellular activity of the Cetux-probe more specifically, confocal microscopy images were acquired over time after treatment with the Cetux-probe for 10 minutes, 4 hours, and 24 hours (Fig. 5b, Fig. 5c, Fig. 5d). After 10 minutes of Cetux-probe treatment, a very faint signal was detected at the surface EGFR on the cell membrane (Fig. 5b, cyan). At 4 hours and 24 hours, the Cetux-probe exhibited strong activated form fluorescence (Fig. 5c, red), with most of it co-localizing with LysoTracker fluorescence (Fig. 5c, green) in lysosomes, producing a rich yellow signal.

The colocalization level between the Cetux-probe and LysoTracker was calculated by analyzing the Pearson correlation coefficient (r), which increased from r = 0.82 at 4 hours to r = 0.92 at 24 hours.

In contrast to the results obtained with the Cetux-probe, the non-quenched Cetux-FPR675 exhibited strong fluorescence at 10 minutes post-treatment. At this time point, the signal was predominantly co-localized with EGFR signals distributed at the cell membrane (Fig. 5b).

Cetux-FPR675 was found to emit fluorescence throughout the entire process from cell membrane entry to lysosomal processing. In contrast, the Cetux-probe primarily exhibited fluorescence within lysosomes, which is thought to be due to proteolytic degradation by lysosomal enzymes.

To measure the intracellular internalization and fluorescence activation of the Cetux-probe in other colorectal cancer cells, KRAS mutant HCT-8 cells (purchased from the Korea Cell Line Bank) were analyzed using the same method as above, yielding results similar to those observed in KRAS wild-type CCK-81 cells (Fig. 5e and Fig. 5f). These results indicate that the internalized Cetux-probe is activated in lysosomes.

### Example 4: Confirmation of Cetux-probe Activation Following Inhibitor Treatment

To further investigate the mechanism of action of the Cetux-probe, the activity of the Cetux-probe was confirmed upon treatment with various inhibitors. The inhibitors used were cetuximab, BafA1, and Z-FA-FMK. Cetuximab was expected to inhibit EGFR-mediated uptake of the Cetux-probe or Cetux-FPR675 by competing for binding to EGFR.

CCK-81 cells were cultured using the same method as in <Example 3>. They were then treated with Cetux-probe or Cetux-FPR675 and either cetuximab (1 mg/mL, coincubated for 4 hours), BafA1 (20 nM, co-treated for 4 hours), or Z-FA-FMK (100 µM, pretreated for 1 hour), followed by nuclear staining with Hoechst solution.

Chloroquine (CQ, 0~100 µM) was used for pretreatment for 3 hours prior to Cetux-probe treatment to inhibit EGFR degradation. Changes in EGFR expression were monitored by Western blot, and the fluorescence intensity of Cetux-probe in cells was evaluated by flow cytometry.

The Western blot procedure for EGFR quantification was as follows: Cells were washed twice with ice-cold PBS before protein extraction, then cell lysates were collected using RIPA buffer containing a 100X protease inhibitor cocktail. Samples were centrifuged at 16,000g for 20 minutes at 4°C, and the supernatant was collected. Protein concentration was measured using the BCA assay, and cell lysates containing equal amounts of protein were loaded onto an 8% SDS-PAGE gel. After electrophoresis at 90 V for 90 minutes, the separated proteins were transferred to a poly(vinylidene fluoride) membrane. The membrane was blocked with 5% (w/v) skim milk at room temperature for 2 hours, then incubated overnight at 4°C using rabbit anti-human EGFR antibody and mouse anti-human GAPDH antibody as primary antibodies.

After washing three times with 1X Tris-buffered saline, HRP-conjugated goat anti-rabbit secondary antibody or HRP-conjugated goat anti-mouse secondary antibody was reacted with the membrane at room temperature for 1 hour. The membrane was washed and treated with an enhanced chemiluminescent solution, and EGFR and GAPDH expression levels were observed using iBright^{™}. EGFR expression levels were quantitatively expressed as relative EGFR levels by calculating the EGFR/GAPDH intensity ratio using Image J software.

Flow cytometry was performed as follows: Cells were dissociated into single cells using trypsin-EDTA, and the fluorescence intensity of the Cetux-probe was analyzed using a CytoFLEX S flow cytometer (Beckman Coulter, USA). Fluorescence data for each sample were quantitatively measured as mean fluorescence intensity (MFI) values from an equal number of cells by gating the live cell population using FlowJo software (BD Bioscience, USA). The MFI of the Cetux-probe was calculated by subtracting the MFI value of the untreated sample from the MFI value of each sample.

As shown in Figs. 6a and 6b, when cells were co-treated with the probe and cetuximab, the fluorescence of the Cetux-probe was inhibited by 85.1±9.1%, and that of Cetux-FPR675 was inhibited by 87.2±5.2%. This indicates that both probes are capable of EGFR-mediated internalization.

Bafilomycin A1 (BafA1), an H+-ATPase inhibitor, is known to inhibit lysosomal degradation by increasing the pH within lysosomes. Cells treated with both the probe and BafA1 showed a 97.4±0.7% inhibition of Cetux-probe fluorescence activation, confirming that Cetux-probe fluorescence is activated by lysosomal degradation.

Furthermore, pretreatment of CCK-81 cells with Z-FA-FMK (Z-Phe-Ala fluoromethyl ketone), a well-known inhibitor of the lysosomal enzyme cathepsin B, resulted in 94.1±2.0% inhibition of Cetux-probe fluorescence activation. In contrast, for Cetux-FPR675, cotreatment with BafA1 or pretreatment with Z-FA-FMK had almost no effect on fluorescence.

Using KRAS mutant HCT-8 cells and performing the experiment in the same manner as above, results similar to those observed in KRAS wild-type CCK-81 cells were observed, as shown in Figs. 6c and 6d. This confirmed that the Cetux-probe is internalized via EGFR binding, activated by lysosomal degradation, and exhibits fluorescence activation.

### Example 5: Monitoring EGFR Degradation

Using Cetux-probe 5-1: Monitoring EGFR Degradation with Cetux-probe in Different Colorectal Cancer Cell Lines We investigated whether the Cetux-probe could monitor EGFR degradation induced by cetuximab.

First, cetuximab (10, 25, 50, and 100 µg/mL) was added to four colorectal cancer cell lines: CCK-81, HCT-8, LoVo, and COLO 320DM. Subsequently, Western blot and EGFR immunohistochemistry analyses were performed using the same methods as in <Example 3> and <Example 4>.

As shown in Figs. 7a and 7b, 100 µg/mL cetuximab significantly promoted EGFR degradation in CCK-81 and HCT-8 cells. However, no EGFR degradation was observed in LoVo or COLO 320DM cells at any of the tested cetuximab concentrations. These results correlate with the fluorescence activation of the Cetux-probe in the four cell lines. CCK-81 and HCT-8 cells exhibited strong fluorescence intensity, whereas LoVo and COLO 320DM cells showed low fluorescence intensity.

Indeed, a significant and strong correlation was observed between cetuximab-induced EGFR degradation and Cetux-probe fluorescence activation in the four cell lines (r = 0.95, P = 0.0498; Fig. 7c).

### 5-2: Monitoring EGFR Degradation with Cetux-probe Following Treatment with Lysosomal Degradation Inhibitors

To further investigate whether the Cetux-probe can monitor EGFR degradation, cetuximab-induced EGFR degradation was suppressed by treating cells with chloroquine (CQ), a lysosomal degradation inhibitor.

In the same manner as in Example 4, HCT-8 cells were respectively treated with CQ (100 µM), BafA1 (100 nM), and Z-FA-FMK (100 µM), followed by treatment with cetuximab (100 µg/mL) for 24 hours. Subsequently, the extent of EGFR degradation was assessed by Western blot.

The results, as shown in Fig. 7d, confirmed that while CQ suppressed cetuximab-induced EGFR degradation in HCT-8 cells, it did not affect EGFR expression in cetuximabuntreated cells. Other lysosomal degradation inhibitors, such as BafA1 and Z-FA-FMK, similarly inhibited cetuximab-induced EGFR degradation in HCT-8 cells (Fig. 7e).

Furthermore, HCT-8 cells were pretreated with various concentrations (0~100 µM) of CQ for 3 hours, followed by treatment with Cetux-probe (1 µM) for 24 hours. The extent of EGFR degradation was then confirmed by Western blot analysis.

As a result, as shown in Fig. 7f, the reduction in EGFR degradation induced by CQ was monitored by changes in the fluorescence intensity of the Cetux-probe. 100µM CQ reduced EGFR degradation by 29.1±4.8% in Western blot analysis and decreased the fluorescence intensity of the Cetux-probe by 86.1±1.1%.

This high sensitivity of the Cetux-probe to EGFR degradation was also observed in the fluorescence signals of cells co-treated with 1, 20, and 50 µM CQ, indicating that the Cetux-probe can be used to measure EGFR degradation in cells.

### Example 6: Confirmation of Cetuximab Drug Responsiveness Using Cetux-probe in Colorectal Cancer Cells

To investigate the relationship between Cetux-probe fluorescence intensity and cetuximab anticancer activity, the present invention used four KRAS wild-type (CCK-81, HT-29, COLO 320DM, and LIM1215) and four KRAS mutant (HCT-8, HCT-15, HCT 116, and LoVo) cell lines.

### 6-1: Confirmation of Cetuximab-Induced Cytotoxicity

First, cytotoxicity following cetuximab treatment was assessed using the Cell Counting Kit-8 (CCK-8) assay.

Each cell line (5000 cells/well) was seeded into a 96-well microplate and treated with various concentrations of cetuximab (0~100 µg/mL), then cultured at 37°C for 48 hours. Absorbance was then measured at 450 nm using a microplate reader. Cell viability for each group (n = 6) was determined relative to untreated cells (set as 100%), and cytotoxicity was calculated as (100 - cell viability)%.

As shown in Fig. 8, CCK-81 cell growth was inhibited by more than 30% at the lowest concentration of cetuximab (0.5 µg/mL), indicating that CCK-81 cells are highly sensitive to cetuximab. Growth of HCT-8 cells was inhibited by more than 20% at cetuximab concentrations ranging from 0.5 µg/mL to 100 µg/mL, indicating that HCT-8 cells are moderately sensitive to cetuximab.

Growth of HCT 116, LIM1215, and HT-29 cells was inhibited by 10-20% at all tested cetuximab concentrations, indicating these cell lines showed moderately low responsiveness to cetuximab.

HCT-15 and LoVo cells were assessed as having low sensitivity to cetuximab, while COLO 320DM cells showed less than 10% growth inhibition at all tested concentrations and were determined to be unresponsive to cetuximab.

### 6-2: Confirmation of Fluorescence Intensity by Cetux-probe

To analyze the fluorescence intensity of the Cetux-probe, the eight cell lines were cultured with 10 nM Cetux-probe for 24 hours, followed by flow cytometry analysis. EGFR degradation levels were confirmed by Western blot.

As shown in Fig. 9a, the results confirmed that the fluorescence intensity of the Cetux-probe was higher in cell lines exhibiting high cytotoxicity to cetuximab.

To confirm their correlation, the fluorescence intensity of the Cetux-probe was compared with the degree of cytotoxicity induced by various concentrations of cetuximab (1, 5, 10, 50, and 100 µg/mL). As shown in Fig. 9b, a strong correlation was confirmed between cetuximab-induced toxicity and Cetux-probe fluorescence intensity.

Among the tested cell lines, only COLO 320DM cells showed a tendency to deviate significantly from the correlation line. This is likely because EGFR expression is extremely low in COLO 320DM cells, potentially limiting the ability of the Cetux-probe to enter these cells.

Four additional KRAS mutant cell lines (SW480, LS513, LS 174T, DLD-1) were added to the eight cell lines mentioned above, for a total of 12 cell lines. The correlation between the fluorescence activity of the Cetux-probe and the cytotoxicity of cetuximab (0.5 µg/mL) was analyzed. As shown in Fig. 9c, the Pearson correlation coefficient remained 0.92 even after adding KRAS mutant cell lines reported to have low cetuximab responsiveness, confirming that the Cetux-probe still exhibits high accuracy for predicting cetuximab treatment efficacy.

### 6-3: Comparison of Cetux-probe with Other Biomarkers

To confirm the superiority of the Cetux-probe in assessing cetuximab drug responsiveness, we analyzed it against other biomarkers employed to predict cetuximab responders and non-responders in colorectal cancer.

First, we evaluated EGFR expression levels in cells, known as a predictive biomarker for response to anti-EGFR antibody therapy. Western blot analysis of EGFR expression in eight colorectal cancer cell lines showed that HCT 116 and LIM1215 cells exhibited high levels of EGFR expression, followed by HT-29, HCT-8, HCT-15, LoVo, and CCK-81 cells, with COLO 320DM cells exhibiting the lowest EGFR expression levels (Fig. 9d, top). No clear linear correlation was observed between initial EGFR expression levels and the degree of cetuximab-induced cytotoxicity (r = 0.24, Fig. 9d bottom).

Furthermore, since KRAS mutation has been established as a biomarker predicting response to cetuximab, the correlation between cetuximab sensitivity and KRAS mutation status was evaluated.

As shown in Fig. 9e, KRAS mutation status demonstrated low predictive ability (P = 0.7549). This discrepancy can be attributed to the fact that while KRAS mutation has been identified as a predictive biomarker for anti-EGFR therapy combined with chemotherapy in metastatic colorectal cancer, in the present invention, KRAS mutation was evaluated as predicting response to anti-EGFR therapy alone. Collectively, these results confirm that the Cetux-probe can be effectively utilized as a powerful tool for predicting responsiveness to cetuximab.

### Example 7: Confirmation of Cetuximab Drug Responsiveness

Using Cetux-probe in Animal Models To investigate the in vivo relevance of the strong correlation observed between Cetux-probe fluorescence activation and in vitro cetuximab cytotoxicity, the present invention used the Cetux-probe to predict responsiveness to cetuximab in xenograft mouse models generated using HCT-8, HT-29, and LoVo cell lines, which represent highly responsive, moderately responsive, and low-responsive cell lines, respectively.

### 7-1: Production of Animal Model and Administration of Probe

BALB/c nu/nu mice were purchased from Orient Bio (Korea), and mice were bred in a germ-free environment at the Korea Institute of Science and Technology (KIST). All live animal experiments were conducted in compliance with relevant laws and institutional guidelines of the Institutional Animal Care and Use Committee (IACUC) at KIST and were approved by the IACUC (Approval No. 2022-04-5052).

To prepare the colorectal cancer xenograft model, HT-29, HCT-8, and LoVo cells were subcutaneously injected into the right flank of BALB/c nu/nu mice at a density of 4 × 10⁶ cells/mouse. In vivo imaging was performed when tumor size reached approximately 160 mm³.

Cetux-FPR675 (15 nmol) and Cetux-probe (15 nmol) were intravenously injected into tumor-bearing mice, and in vivo imaging was performed using IVIS (PerkinElmer, USA) at 660 nm excitation and 710 nm emission for 7 days. Subsequently, three mice from each group were sacrificed, and the heart, lungs, liver, spleen, kidneys, and tumor were extracted for ex vivo imaging.

Fluorescence intensity was measured as the average radiant efficiency using Living Image software (PerkinElmer, USA). The fluorescence radiant efficiency for the tumor was calculated as a percentage by dividing the fluorescence intensity of the isolated tumor by the total fluorescence intensity from the tumor and other organs combined.

To determine the optimal time for fluorescence visualization after Cetux-probe administration, mice bearing HT-29 colorectal cancer were intravenously injected with Cetux-probe. Fluorescence signals were observed at various time points. Fluorescence signals predominantly appeared in the tumor starting 16 hours post-injection (Fig. 10a and Fig. 10b). The fluorescence signal rapidly increased to a peak on day 1 after Cetux-probe injection and then slowly decreased until day 7 post-injection, whereas the Cetux-FPR675 signal developed very rapidly up to 2 hours post-injection. Furthermore, the activatable Cetux-probe exhibited more concentrated fluorescence at the tumor site compared to the "always-on" Cetux-FPR675 in vivo.

Ex vivo analysis further confirmed that mice treated with the Cetux-probe (37.2 ± 2.5%) exhibited higher fluorescence radiant efficiency toward tumors compared to those treated with Cetux-FPR675 (26.1 ± 3.1%, P < 0.01; Fig. 10c).

### 7-2: Cetuximab Treatment in Mice with Tumors

Mice administered with Cetux-probe using the same method as in <Example 7-1> were monitored using an IVIS imaging system (Fig. 11a). After imaging completion, mice received intraperitoneal injections of cetuximab at a dose of 25 mg/kg every 3 days for a total of 8 doses. Tumor size was measured every 2 days using calipers, and tumor volume was calculated as length × (width)² × 0.53 (control group n=3, cetuximab-treated group n=5).

The day after all treatments were completed, the tumors were extracted, and a portion of each tumor was used to prepare lysates for Western blot and paraffin blocks for immunohistochemistry.

Specifically, for immunohistochemical staining for EGFR, the excised tumors were fixed in 4% paraformaldehyde, and tissue sections were obtained from the paraffin blocks. The slides were incubated at 60°C for 1 hour, then immersed in xylene for paraffin removal. Subsequently, the slides were immersed in 100% to 70% ethanol, rinsed with distilled water to rehydrate, treated with antigen retrieval buffer for 20 minutes, and blocked with 1% (w/v) BSA/PBS buffer for 30 minutes. Primary staining was then performed overnight at 4°C with anti-EGFR antibody. The next day, secondary staining was performed at room temperature for 1 hour with Alexa Fluor 488-labeled goat anti-rabbit antibody, followed by nuclear staining. EGFR expression was quantified using confocal microscopy based on Alexa Fluor 488 fluorescence intensity.

As a result, as shown in Fig. 11b, tumor size was nearly identical in HCT-8, HT-29, and LoVo xenograft mice when imaged after Cetux-probe administration (HCT-8: 166.46 ± 15.42 mm³; HT-29: 166.79 ± 9.74 mm³; and LoVo: 165.66 ± 17.46 mm³).

Quantification of the average radiant efficiency of Cetux-probe in the in vivo tumor regions at 8, 16, 24, and 32 hours after administration revealed that the fluorescence intensity of Cetux-probe in the HCT-8 tumors, as shown in Fig. 11c, Fig. 11d, and Fig. 11e (1.34±0.07×10¹⁰ at 32 hours) was significantly higher than that in mice with HT-29 tumors (1.13±0.07×10¹⁰ at 32 hours) and mice with LoVo tumors (0.95±0.06×10¹⁰ at 32 hours).

Following treatment of mice with cetuximab, the degree of tumor suppression was observed. As shown in Fig. 11f, cetuximab significantly inhibited tumor growth in mice with HCT-8 tumors and mice with HT-29 tumors compared to untreated control mice. In contrast, in mice bearing LoVo tumors, cetuximab only slightly inhibited tumor growth, with no significant difference in tumor volume between cetuximab-treated and untreated control mice.

Next, to determine whether the growth-inhibitory effect of cetuximab was related to EGFR degradation, tumors were resected the day after all treatments were completed, and EGFR levels were assessed by Western blot and immunohistochemistry.

The results showed that, as depicted in Fig. 11g and Fig. 11h, EGFR levels in HCT-8 tumors were significantly reduced by cetuximab compared to LoVo tumors. This was further evidenced by the higher fluorescence intensity of the Cetux-probe in HCT-8 tumor tissue compared to LoVo tumor tissue. Overall, the results of the present invention indicate that the fluorescence intensity of the Cetux-probe in tumors correlates with the degree of EGFR degradation and the therapeutic efficacy of cetuximab in mice with colorectal cancer.

### [INDUSTRIAL APPLICABILITY]

The cetuximab-conjugated probe (Cetux-probe) of the present invention operates via the same mechanism as cetuximab therapy, enabling rapid and accurate prediction of cetuximab's therapeutic efficacy and responsiveness. The activated fluorescence of the Cetux-probe can be used to measure EGFR degradation, and a strong linear correlation was observed between the fluorescence and the cytotoxicity of cetuximab in colorectal cancer cells and in mice bearing tumors. Furthermore, it was confirmed that the predictive capability of the activated fluorescence of the Cetux-probe is significantly higher than that of EGFR expression or KRAS mutation status. Therefore, the Cetux-probe of the present invention can be effectively utilized to predict responsiveness to cetuximab therapy by evaluating EGFR degradation.

## Claims

1. A composition for predicting cetuximab responsiveness or confirming the prognosis regarding cetuximab treatment efficacy, comprising a complex, wherein the complex comprises: a peptide represented by the amino acid sequence of SEQ ID NO: 1, which is cleaved by a lysosomal enzyme present in a cancer cell; a fluorophore and a quencher bound to the peptide; and cetuximab bound to the C-terminus of the peptide.

2. The composition of Claim 1, wherein the fluorophore is bound to the N-terminus of the peptide, and the quencher is bound to the epsilon amine group of a lysine or arginine amino acid residue of the peptide.

3. The composition of Claim 1, wherein the fluorophore is selected from the group consisting of fluorescein, FITC (fluorescein isothiocyanate), Oregon green, Texas red, Cy2, Cy3, Cy3B, Cy3.5, Cy5, Cy5.5, Cy7, indocarbocyanine, rhodamine, oxacarbocyanine, thiacarbocyanine, merocyanine, pyrodyloxazole, nitrobenzoxadiazole, benzoxadiazole, Nile red, Nile orange, acridine yellow, aumarine, crystal violet, and malachite green.

4. The composition of Claim 1, wherein the quencher is selected from the group consisting of TAMRA (6-carboxytetramethylrhodamine), BHQ1 (black hole quencher 1), BHQ2 (black hole quencher 2), BHQ3 (black hole quencher 3), NFQ (nonfluorescent quencher), dabcyl, Eclipse, DDQ (Deep Dark Quencher), Blackberry Quencher, and Iowa Black.

5. The composition of Claim 1, wherein the cancer is characterized as epidermal growth factor receptor (EGFR)-positive colorectal cancer.

6. The composition of Claim 1, wherein the complex can penetrate cancer cells and be cleaved by enzymes present within the lysosomes of the cancer cells, thereby relieving the quenching effect of the quencher on the fluorophore and allowing fluorescence to be emitted, and wherein a higher fluorescence intensity indicates higher responsiveness to cetuximab or a higher therapeutic efficacy of cetuximab.

7. A method for providing information to predict responsiveness to cetuximab or therapeutic efficacy of cetuximab, comprising the steps of treating a sample isolated from a cancer patient with the composition according to any one of Claims 1 to 6, and measuring the fluorescence intensity.

8. The method of Claim 7, wherein if the fluorescence intensity is detected, the cancer patient is determined to have responsiveness to cetuximab or to have therapeutic efficacy from cetuximab.

9. A method for screening an anticancer drug having EGFR degradation activity, comprising the steps of: treating an EGFR-positive cancer cell line, which has been pretreated with the composition according to any one of claims 1 to 6, with a candidate substance; and measuring the fluorescence intensity generated by the composition.

10. A composition for diagnosing EGFR-positive cancer, comprising a complex, wherein the complex comprises: a peptide represented by the amino acid sequence of SEQ ID NO: 1, which is cleaved by a lysosomal enzyme present in a cancer cell; a fluorophore and a quencher bound to the peptide; and cetuximab bound to the C-terminus of the peptide.
